# EUROPEAN PATENT SPECIFICATION

(11) **EP 3 658 490 B1**
(45) Date of publication and mention of the grant of the patent: **03.07.2024**
(21) Application number: 18739540.5
(22) Date of filing: 11.07.2018
(51) Int. Cl.: C01B 3/02, C01B 3/38, C01C 1/04, C01B 13/02, C25B 1/04, C01B 3/48, C01B 3/58

(54) **METHOD FOR IMPROVING EFFICIENCY OF AN AMMONIA SYNTHESIS GAS PLANT**
VERFAHREN ZUR VERBESSERUNG DER EFFIZIENZ EINER AMMONIAKSYNTHESEGASANLAGE
PROCÉDÉ D'AMÉLIORATION DE L'EFFICACITÉ D'UNE INSTALLATION DE GAZ DE SYNTHÈSE D'AMMONIAC

(30) Priority: 25.07.2017 DK PA201700425; 25.09.2017 DK PA201700522
(43) Date of publication of application: 03.06.2020
(73) Proprietor: Topsoe A/S, 2800 Kgs. Lyngby (DK)
(72) Inventor: HAN, Pat A., 2765 Smørum (DK); KRØLL JENSEN, Annette E., 3480 Fredensborg (DK)
(74) Representative: Topsoe A/S
(86) International application number: PCT/EP2018/068806
(87) International publication number: WO 2019/020377

(56) References cited:
- WO-A1-2016/149507
- CN-A- 101 892 492
- JP-A- 2011 132 103
- US-A1- 2009 165 459
- US-A1- 2012 100 062
- US-A1- 2013 345 325

## Description

The present application is directed to the preparation of ammonia synthesis gas. More particular, the invention is a method for improving efficiency of a conventional ammonia synthesis gas plant by combining electrolysis of water and the conventional primary and secondary steam reforming of a hydrocarbon feed stock for the preparation of hydrogen and nitrogen containing ammonia synthesis gas.

Ammonia synthesis gas is conventionally prepared by subjecting hydrocarbon feed typically natural gas and/or higher hydrocarbons to endothermic steam reforming reactions in a fired tubular primary steam reformer by contact with a steam reforming catalyst. The primary reformed gas is then fed into a secondary adiabatic steam reformer, wherein part of hydrogen formed in the primary steam reforming and residual amounts of hydrocarbons in the gas from the primary steam reforming are partial oxidized with air and steam and subsequently reformed in presence of a secondary reforming catalyst. From the secondary reformer, raw synthesis gas is withdrawn containing hydrogen, carbon monoxide and carbon dioxide formed during reaction of the feedstock in the above steam reforming reactions and nitrogen introduced into the gas through addition of air in the secondary reforming step.

The disadvantage of the primary and secondary reforming process is a relatively high hydrocarbon feed stock and fuel consumption for use in heating the endothermic primary steam reforming in the fired primary steam reformer and consequently a large CO₂ emission in the flue gas from burners used to heat the reformer. The CO₂ product can be captured from the process and used for downstream processes such as urea production or enhanced oil recovery.

However, primary and secondary steam reforming is still frequently employed in the industry, particularly in existing reforming plants for the production of ammonia synthesis gas.

Secondary steam reforming comprises partial oxidation, using oxygen containing atmosphere, of a primary reformed feed gas to CO, CO₂, H₂, H₂O and remaining hydrocarbon and subsequently steam reforming of the hydrocarbon to form raw synthesis gas.

WO 2016/149507 discloses a process and a plant for the preparation of ammonia. The process and the plant comprises first reforming in a steam reformer and subsequently in a secondary reformer. Air and oxygen is added to the secondary reformer. The reformed gas is then treated in a water gas shift section, a separation unit and in a methanation reactor before it is passed via a syngas compressor to an ammonia synthesis module.

US 2013/345325 mentions use of oxygen from electrolysis of water in an autothermal reactor for the production of synthesis gas.

Recently, a combination of electrolysis of water for production of hydrogen and air separation for the production of nitrogen has been envisaged for the preparation of ammonia synthesis gas, at least in patent literature. The thus produced hydrogen and nitrogen are combined in stoichiometric ratios to form synthesis gas for ammonia production. The disadvantage of the combination of electrolysis and air separation is, however, that oxygen is produced as by-product in both electrolysis and air separation, which has no use in the ammonia synthesis, and can be considered as energy loss.

Typically, existing industrial ammonia synthesis gas plants, the so-called front end of an ammonia plant comprise as already mentioned above, a fired primary steam reformer, a secondary steam reformer with a burner at gas inlet side and a steam reforming catalyst bed at gas outlet side. The burner is typically operated with air.

The raw ammonia synthesis gas withdrawn from the secondary steam reformer is subsequently treated in a water gas shift unit for the production of further hydrogen and conversion of carbon monoxide to carbon dioxide by the known water gas shift reaction.

The carbon dioxide contained in the shifted ammonia synthesis gas is then removed in a carbon dioxide removal process.

Remaining amounts of carbon dioxide and/or carbon monoxide in the ammonia synthesis gas from the carbon dioxide removal process are removed by methanation in a chemical reaction that converts carbon monoxide and/or carbon dioxide to methane.

The thus prepared ammonia synthesis gas is introduced into an ammonia make up gas compressor and sent into the ammonia production unit.

The present invention is based on establishing a combination of the fired primary steam reforming process and the secondary reforming process using air or oxygen enriched air in the operation of the secondary reformer burner and a new implemented step of electrolysis of water for the production of ammonia synthesis gas.

Thus, this invention provides method of improving efficiency of an ammonia synthesis gas plant, the ammonia synthesis gas plant comprises a fired primary steam reformer and a secondary steam reformer operated with an oxygen containing atmosphere, a water gas shift unit, a carbon dioxide removal unit, a methanation step and an ammonia synthesis gas compressor, the method comprises the steps of
(a) establishing an electrolysis unit and preparing a separate hydrogen gas containing stream and a separate oxygen gas containing stream by electrolysis of water;
(b) establishing a gas pipe for transporting the separate hydrogen gas containing stream from the electrolysis unit to the synthesis gas compressor and/or to the methanation step; and
(c) establishing a gas pipe for transporting at least part of the separate oxygen gas stream from the electrolysis unit to a burner in the secondary reformer.

The method of the invention can be used to improve efficiency of an existing ammonia synthesis gas plant operated with primary and secondary reforming or in a new plant with primary and secondary reforming. The improvement of an existing or a new ammonia synthesis gas plant by the method of the invention aims to increase the production capacity of the plant and/or to save fuel in the fired primary steam reformer at a fixed capacity, as oxygen from water electrolysis provides heat for the reforming reaction in the secondary reformer. Thereby, the duty of the primary reformer is decreased, when the oxygen content in the oxygen containing atmosphere in the secondary reformer is increased with the oxygen prepared in the water electrolysis. As a result, the hydrocarbon slip in the gas from the primary reformer increases and the gas exit temperature decreases, which again results in lower fuel consumption for firing the primary reformer. Due to the lower fuel consumption, the reformer tube wall temperature is reduced, resulting in a significantly longer tube life time.

Another advantage is that the overall hydrocarbon slip outlet the secondary reformer can be the same as in conventional plants without electrolysis or can be reduced to obtain improved synthesis gas composition because of reduced content of inerts resulting in reduced purge from the ammonia loop and thus a more efficient utilization of the feed stock.

The method according to the invention provides further advantage of less emission of CO₂ from the primary flue gas stack.

Still an advantage is that the CO₂ partial pressure is increased at inlet to the carbon dioxide removal unit, which improves the carbon dioxide removal efficiency by reducing the required energy consumption.

Compared to prior art methods using electrolysis of water for hydrogen production and air separation for nitrogen production, the oxygen product from electrolysis of water is advantageously used for partial oxidation in secondary reformer resulting in a reduced size of the primary reformer in a new plant or reduced load in an existing plant, which is a costly and an energy intensive unit and process.

Still an advantage of the invention is that energy for operating the electrolysis unit can be renewable energy generated by windmills, solar cells, hydraulic energy or other renewables.

Thus, in a preferred embodiment of the invention, the electrolysis unit is powered by renewable energy.

Preferably, the electrolysis of water is performed at elevated pressure according to process air compressor discharge pressure, which delivers the prepared stream of oxygen at elevated pressure to the burner of the secondary reformer and the hydrogen stream to the synthesis gas compressor and/or to the methanation step.

Thus, in a preferred embodiment of the invention, the electrolysis unit is pressurized.

The synergy in combining water electrolysis with secondary reforming technology for ammonia synthesis gas production, results in overall savings of hydrocarbon feedstock and fuel for the reforming process.

In Table 1 below, key figures of ammonia synthesis gas preparation are given for a 2200 MTPD ammonia plant for comparison of conventional syngas technologies and conventional syngas technology combined with water electrolysis.

**Table 1**

| Technology for syngas preparation | Natural gas feed consumption, Nm³/h | Natural gas fuel consumption, Nm³/h | Power for electrolysis, MW | CO₂ in flue gas, Nm³/h | Primary reformer duty, Gcal/h | Tₒᵤₜ Primary Reformer, °C |
|---|---|---|---|---|---|---|
| Conventional | 57,408 | 19,273 | 0 | 21,899 | 108.82 | 807 |
| Conventional with water electrolysis (25% oxygen in air) | 57,108 | 14,072 | 54 | 16,438 | 82.34 | 748 |

## Claims

1. A method of improving efficiency of an ammonia synthesis gas plant, the ammonia synthesis gas plant comprises a fired primary steam reformer and a secondary steam reformer operated with an oxygen containing atmosphere, a water gas shift unit, a carbon dioxide removal unit, a methanation step and an ammonia synthesis gas compressor, the method comprises the steps of
(a) establishing an electrolysis unit and preparing a separate hydrogen gas containing stream and a separate oxygen gas containing stream by electrolysis of water;
(b) establishing a gas pipe for transporting the separate hydrogen gas containing stream from the electrolysis unit to the synthesis gas compressor and/or to the methanation step; and
(c) establishing a gas pipe for transporting at least part of the separate oxygen gas stream from the electrolysis unit to a burner in the secondary reformer.

2. The method according to claim 1, wherein the electrolysis unit is powered by renewable energy.

3. The method according to claim 1 or 2, wherein the oxygen containing atmosphere, is air enriched with oxygen from the separate oxygen gas stream.

4. The method according to any one of claims 1 to 3, wherein the electrolysis unit is pressurized.

5. Improved ammonia synthesis gas plant comprising a fired primary steam reformer and a secondary steam reformer operated with an oxygen containing atmosphere, a water gas shift unit, a carbon dioxide removal unit, a methanation reactor and an ammonia synthesis gas compressor, wherein the ammonia synthesis gas plant further comprises an electrolysis unit providing a separate hydrogen containing stream and a separate oxygen gas containing stream by electrolysis of water and a gas pipe for transporting the separate hydrogen gas containing stream from the electrolysis unit to the synthesis gas compressor and/or to the methanation reactor and a gas pipe for transporting at least part of the separate oxygen gas stream from the electrolysis unit upstream or into a burner in the secondary reformer.

## Patentansprüche

1. Verfahren zur Verbesserung der Effizienz einer Ammoniak-Synthesegasanlage, wobei die Ammoniak-Synthesegasanlage einen befeuerten primären Dampfreformer und einen sekundären Dampfreformer, die mit einer sauerstoffhaltigen Atmosphäre betrieben werden, eine Wassergas-Shift-Einheit, eine Kohlendioxid-Entfernungseinheit, einen Methanisierungsschritt und einen Ammoniak-Synthesegaskompressor umfasst, wobei das Verfahren die Schritte umfasst, zum
(a) Einrichten einer Elektrolyseeinheit und Herstellen eines separaten wasserstoffgashaltigen Stroms und eines separaten sauerstoffgashaltigen Stroms durch Elektrolyse von Wasser;
(b) Einrichten einer Gasleitung zum Transportieren des separaten wasserstoffgashaltigen Stroms von der Elektrolyseeinheit zum Synthesegaskompressor und/oder zum Methanisierungsschritt; und
(c) Einrichten einer Gasleitung zum Transportieren mindestens eines Teils des separaten Sauerstoffgasstroms von der Elektrolyseeinheit zu einem Brenner im sekundären Reformer.

2. Verfahren nach Anspruch 1, wobei die Elektrolyseeinheit mit erneuerbarer Energie betrieben wird.

3. Verfahren nach Anspruch 1 oder 2, wobei die sauerstoffhaltige Atmosphäre mit Sauerstoff aus dem separaten Sauerstoffgasstrom angereicherte Luft ist.

4. Verfahren nach einem der Ansprüche 1 bis 3, wobei die Elektrolyseeinheit unter Druck steht.

5. Verbesserte Ammoniak-Synthesegasanlage, umfassend einen befeuerten primären Dampfreformer und einen sekundären Dampfreformer, die mit einer sauerstoffhaltigen Atmosphäre betrieben werden, eine Wassergas-Shift-Einheit, eine Kohlendioxid-Entfernungseinheit, einen Methanisierungsreaktor und einen Ammoniak-SynthesegasKompressor, wobei die Ammoniak-Synthesegasanlage weiter eine Elektrolyseeinheit, die einen separaten wasserstoffhaltigen Strom und einen separaten sauerstoffgashaltigen Strom durch Elektrolyse von Wasser bereitstellt, und eine Gasleitung zum Transportieren des separaten wasserstoffgashaltigen Stroms von der Elektrolyseeinheit zum Synthesegaskompressor und/oder zum Methanisierungsreaktor und eine Gasleitung zum Transportieren mindestens eines Teils des separaten Sauerstoffgasstroms von der Elektrolyseeinheit stromaufwärts oder in einen Brenner im Sekundärreformer umfasst.

## Revendications

1. Procédé d'amélioration de l'efficacité d'une installation de gaz de synthèse d'ammoniac, l'installation de gaz de synthèse d'ammoniac comprenant un reformeur à vapeur primaire alimenté et un reformeur à vapeur secondaire fonctionnant avec une atmosphère contenant de l'oxygène, une unité de conversion de gaz à l'eau, une unité d'élimination de dioxyde de carbone, une étape de méthanation et un compresseur de gaz de synthèse d'ammoniac, le procédé comprend les étapes suivantes
(a) mise en place d'une unité d'électrolyse et préparation d'un flux séparé contenant de l'hydrogène gazeux et d'un flux séparé contenant de l'oxygène gazeux par électrolyse de l'eau ;
(b) mise en place d'un tuyau de gaz pour transporter le flux séparé contenant de l'hydrogène gazeux depuis l'unité d'électrolyse vers le compresseur de gaz de synthèse et/ou vers l'étape de méthanisation ; et
(c) mise en place d'un tuyau de gaz pour transporter au moins une partie du flux séparé d'oxygène gazeux depuis l'unité d'électrolyse vers un brûleur dans le reformeur secondaire.

2. Procédé selon la revendication 1, dans lequel l'unité d'électrolyse est alimentée par une énergie renouvelable.

3. Procédé selon la revendication 1 ou 2, dans lequel l'atmosphère contenant de l'oxygène est de l'air enrichi en oxygène provenant du flux séparé d'oxygène gazeux.

4. Procédé selon l'une quelconque des revendications 1 à 3, dans lequel l'unité d'électrolyse est sous pression.

5. Installation de gaz de synthèse d'ammoniac améliorée comprenant un reformeur à vapeur primaire alimenté et un reformeur à vapeur secondaire fonctionnant avec une atmosphère contenant de l'oxygène, une unité de conversion de gaz à l'eau, une unité d'élimination de dioxyde de carbone, un réacteur de méthanation et un compresseur de gaz de synthèse d'ammoniac, dans laquelle l'installation de gaz de synthèse d'ammoniac comprend en outre une unité d'électrolyse fournissant un flux séparé contenant de l'hydrogène et un flux séparé contenant de l'oxygène gazeux par électrolyse de l'eau et un tuyau de gaz pour transporter le flux séparé contenant de l'hydrogène gazeux depuis l'unité d'électrolyse vers le compresseur de gaz de synthèse et/ou vers le réacteur de méthanation et un tuyau de gaz pour transporter au moins une partie du flux séparé d'oxygène gazeux depuis l'unité d'électrolyse en amont ou dans un brûleur dans le reformeur secondaire.
